(19) Europäisches Patentamt / European Patent Office / Office européen des brevets

(11) **EP 3 403 205 B1**

(12) **EUROPEAN PATENT SPECIFICATION**

(45) Date of publication and mention
of the grant of the patent:
**31.12.2025 Bulletin 2026/01**

(21) Application number: **17702762.0**

(22) Date of filing: **12.01.2017**

(51) International Patent Classification (IPC):
***G16C 20/30*** *(2019.01)*

(52) Cooperative Patent Classification (CPC):
**G16C 20/30**

(86) International application number:
**PCT/EP2017/000033**

(87) International publication number:
**WO 2017/121641 (20.07.2017 Gazette 2017/29)**

(54) **ANALYTIC GIBBS-DUHEM CORRECTION FOR QUASI-CHEMICAL MODELS WITH COMPOSITION DEPENDENT INTERACTIONS**

ANALYTISCHE GIBBS-DUHEM-KORREKTUR FÜR QUASI-CHEMISCHE MODELLE MIT ZUSAMMENSETZUNGSABHÄNGIGEN WECHSELWIRKUNGEN

CORRECTION DE GIBBS-DUHEM ANALYTIQUE POUR DES MODÈLES QUASI-CHIMIQUES AYANT DES INTERACTIONS DÉPENDANT DE LA COMPOSITION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.01.2016 DE 102016000297**

(43) Date of publication of application:
**21.11.2018 Bulletin 2018/47**

(73) Proprietor: **Dassault Systèmes Americas Corp.
02451-1223, Waltham, Massachusetts (US)**

(72) Inventor: **KLAMT, Andreas
51379 Leverkusen (DE)**

(74) Representative: **Bandpay & Greuter
11 rue Christophe Colomb
75008 Paris (FR)**

(56) References cited:
**WO-A1-2012/051242**

• **KLAMT A. ET AL: "COSMOSPACE: Alternative to Conventional Activity-Coefficient Models", AICHE JOURNAL, vol. 48, no. 10, 1 October 2002 (2002-10-01), US, pages 2332 - 2349, XP055365271, ISSN: 0001-1541, DOI: 10.1002/ aic.690481023**
• **KLAMT A.: "COSMO-RS for aqueous solvation and interfaces", FLUID PHASE EQUILIBRIA, vol. 407, 23 May 2015 (2015-05-23), pages 152 - 158, XP029290782, ISSN: 0378-3812, DOI: 10.1016/ J.FLUID.2015.05.027**

**Description**

The invention relates to a method for selecting solvents for a reaction step using a computer-implemented method for simulation of at least one physical property of a system of one or more chemical species that includes at least one chemical species dissolved in at least one chemical species, using a quasi-chemical, COSMO-RS, COSMO-SAC, or COSMOSPACE calculation involving composition dependent segment interactions and achieving Gibbs-Duhem consistent chemical potentials and activity coefficients or Gibbs-Duhem consistent chemical potentials or Gibbs-Duhem consistent activity coefficients.

FIELD OF THE INVENTION

The conductor-like screening model for realistic solvation (COSMO-RS) was introduced 20 years ago and meanwhile has become an important tool for the prediction of fluid phase equilibrium properties. Starting from quantum chemical information about the surface polarity of solutes and solvents, it solves the statistical thermodynamics of molecules in liquid phases by the extremely efficient approximation of independently pair-wise interacting surfaces, which meanwhile was shown to be equivalent to Guggenheim's quasi-chemical theory. One of the basic limitations of COSMO-RS, as of any quasi-chemical model, is the neglect of neighbor information, i.e. of local correlations of surface types on the molecular surface.

BACKGROUND OF THE INVENTION

The conductor-like screening model for realistic solvation (COSMO-RS) [1] [2] [3] originally presented in 1995 starts from quantum-chemical calculations of molecules embedded in a virtual conductor, employing the conductor-like screening model (COSMO) [4]. In a second step these polarization charge densities are used for the quantification of the intermolecular electrostatic and hydrogen-bond interactions. In a final step these local surface interaction energies are turned into free energies and enthalpies of fluids, using a specially derived thermodynamic self-consistency equation, which later was published in a slightly more general form under the name COSMOSPACE (COSMO surface pair activity coefficient equations) [5]. COSMO-RS meanwhile has become one of the most important and accurate tools for the prediction of free energies of solvation and important fluid phase equilibrium properties as solubilities, vapor pressures, activity and partition coefficients [6].

A few years after the publication of COSMO-RS it was shown in K. Lucas, RWTH Aachen, personal communication 2001 [7] that the thermodynamic model of interacting surface segments derived in COSMO-RS directly from statistical thermodynamics, i.e. COSMOSPACE, is equivalent to an efficient and exact solution of Guggenheim's quasi-chemical theory (QUAC) shown in E.A. Guggenheim, Mixtures, Oxford University Press, Oxford, 1952 [8], directly yielding Gibbs-Duhem consistent activity coefficients. Usually the QUAC equations are either solved by the less efficient variational optimization of all contact probabilities, as in GEQUAC [9], or by additional approximations as in UNIQUAC [10] and UNIFAC [11]. In 1986 Larsen and Rasmussen [12] had published an algorithm for solving the quasi-chemical equation systems, which is completely equivalent to the COSMOSPACE equations, but they did not recognize that these equations directly lead to free energies and activity coefficients. In 2001 Lin and Sandler re-derived the COSMO-RS algorithm under the name COSMO-SAC (COSMO segment activity coefficients) [13].

Despite its considerable success, one of the fundamental limitations of COSMO-RS, as of all quasi-chemical models, is the almost complete neglect of effects resulting from the spatial neighborhood of different segments on the molecular surface. For example, the dimerization of acetic acid diluted in non-polar solvents is out of reach for such models, because the hydrogen bond donors and acceptors of the carboxylic acid group would form individual strong contacts, leading to a 4th power dependence of the dimer concentration with respect to the concentration of the acid, while in reality the first contact between two acid molecules will induce a high probability for the formation of the second hydrogen bond, which then leads to a 2nd order concentration dependence of the dimer concentration. Indeed, recently an extension of COSMO-RS for dimerization, association, and reaction equilibria (COSMO-RS-DARE) has been presented [14], which is able to describe such phenomena if the dimers or association species are introduced as special species into the COSMO-RS ensemble. But this extension requires considerable special parameterization and it is not extendable to other, less specific surface correlation phenomena. Other cases in which the lack of surface correlation may cause inaccuracies in COSMO-RS are micro phase separations, as they are observed in amphiphilic liquids as octanol, in which the alkyl chains and the polar groups tend to preferentially cluster, even before micellization occurs. This is that there have been no further developments to correct the error by accepting the independent parts of the molecule.

Object of the invention is to improve a method which makes it possible to calculate and to simulate the activity coefficient and related properties of substances in solution and to interpret this data by means of chemical methods suitable to select suitable solvents for a reaction step.

This problem is solved according to the invention by all the features of the independent process claim 1 and by all the features of the independent product claim 9. The dependent patent claims specify possible embodiments.

The invention is a method for selecting solvents for a reaction step using a computer-implemented method for simulation of at least one physical property of a system of one or more chemical species that includes at least one chemical species dissolved in at least one chemical species, using a second quasi-chemical, COSMO-RS, COSMO-SAC, or COSMOSPACE calculation involving composition dependent segment interactions $g_{\mu\nu}$, and achieving Gibbs-Duhem consistent chemical potentials and activity coefficients or Gibbs-Duhem consistent chemical potentials or Gibbs-Duhem consistent activity coefficients, characterized in that segment contacting probabilities, resulting from a first quasi-chemical, COSMO-RS, COSMO-SAC or COSMOSPACE calculation for quantifying the induced local concentrations of surface types or polarization charge densities due to the neighbor segments of contacting neighbor segments, are used as an input variable in the second calculation, the second calculation thereby resulting in modified segment contact probabilities which reflect the local correlations of surface polarities, wherein the induced local concentrations are converted into an entropic correction for the pair-wise interaction of segments and the second calculation uses the local concentration corrected interaction energies.

## DETAILED DESCRIPTION OF THE PREFERRED EMBODIMENT

**The invention generally is directed** to **a** method for selecting solvents for a reaction step using a computer-implemented method for simulation as defined in claim 1.

In detail, the present invention relates to the use of composition dependent interaction parameters in COSMO-RS or other fluid phase thermodynamic models of the class of quasi-chemical models such as COSMO-SAC or COSMOSPACE calculation. Usually such models operate which interaction parameters describing the interactions between each combination of surface types, which only depend on the surface types, but not on the overall composition of the simulated liquid system. In that case the chemical potentials of the simulated liquid species can be directly and very efficiently calculated from recursive equations presented by Klamt et al. as COSMO-RS or COSMOSPACE equations. Larsen and Rasmussen had earlier suggest an equivalent equation without recognizing the value of the method the direct calculation of chemical potentials. Lin and Sandler have presented the COSMOSPACE equations slightly reformulated, but equivalent, under the name COSMO-SAC. The direct calculation of chemical potentials from COSMOSPACE and similar equations fails, as soon as composition dependent interaction parameters are used. The resulting quantities would be no more Gibbs-Duhem consistent and thus invalid chemical potentials. The invention describes a correction to the COSMOSPACE or similar equations, which conserves the Gibbs-Duhem consistency in the case of composition dependent parameters and thus allows for many extensions and improvements of quasi-chemical models, which lead to higher accuracy and braoder applicability of such models while conserving the ability to efficiently derive Gibbs-Duhem consistent chemical potentials.

A special case of involving such composition dependence is a completely novel concept of using the first order COSMO-RS or other fluid phase thermodynamic models of the class of quasi-chemical models contact probabilities as for the construction of local surface correlation functions. These are fed as an entropic correction in form of input variables resulting from an initial quasi-chemical, COSMO-RS, COSMO-SAC or COSMOSPACE calculation as for the pair interactions for example into a second COSMO-RS self-consistency loop, which yields new contact probabilities, enthalpies, free energies and activity coefficients recovering much of the originally lost neighbor effects, mentioned as COSMO-RSC. By this novel analytic correction for concentration dependent interactions the resulting activity coefficients are exactly Gibbs-Duhem consistent. The theory is demonstrated in the method of the present invention on the example of a lattice Monte-Carlo fluid of dimerizing pseudo-molecules. In this example the strong deviations of the lattice Monte-Carlo fluid from quasi-chemical theory are almost perfectly reproduced by COSMO-RSC without any adjustable parameter.

In a preferred embodiment of the invention, the novel concept of the present invention can be performed using a computer-implemented simulation taking into account local correlations of surface polarities in a much more systematic and almost parameter-free way. This 2nd order COSMO-RS, which is denoted as COSMO-RSC further on, uses the contact probabilities of surface segments derived in a first COSMO-RS cycle for the quantification of for example the induced local concentrations of surface types or polarization charge densities due to the neighbor segments of contacting neighbor segments. For example, these induced local concentrations then can be converted into an entropic correction for the pair-wise interaction of segments. Then a second COSMO-RS loop is performed, for example utilizing the local concentration corrected interaction (free) energies, resulting in modified contact probabilities, which reflect the local correlations. This easily leads to corrected interaction enthalpies of the liquid ensemble, while more theory is required in order to get thermodynamically consistent free energies. This is because the exemplary use of concentration dependent interaction operators, as the concentration dependent correlation corrections, in the COSMOSPACE algorithm lead to Gibbs-Duhem inconsistent free energies and activity coefficients. A theoretical analysis and a resulting correction for this problem has already been developed some time ago. Using this correction, the simulation method of the present invention ends up in COSMO-RSC with exactly Gibbs-Duhem consistent activity coefficients.

The present invention is further described in detail with respect to the accompanying method, the accompanying example and the drawings. Features discussed within the method, within the example and features discussed within the drawings and in the description of the drawings can be combined with each other freely. The features mentioned in the claims and the specification are essential to the invention, either in themselves or in any given combination with the features discussed within the method, the example and the drawings and in the description of the drawings.

METHOD

To demonstrate and to test the new calculating/simulation method an artificial lattice Monte-Carlo (LMC) liquid is used, which allows for a clearer analysis of the local surface correlations than real fluids would do. Using the notations introduced in the COSMOSPACE paper [5], an ensemble of molecules that represents a liquid is considered. The combinatorial free energy is neglected, since it is not of relevance for the aspects considered here, and only analyze the so-called "residual" contribution, which arises from the interactions between molecules. The basic assumption of surface pair interaction models is that residual - i.e., non-steric - interactions can be described by pairs of geometrically independent surface segments.

A system having $v$ different types of surface considered. The total number of surface segments $n_i$ on a molecule $i$ is given by

$$n_i = \sum_v n_i^v = \frac{q_i}{a_{eff}} \tag{1}$$

where $q_i$ is the total surface area of molecule $i$ and $n_i^v$ denotes the numbers of segments of type $v$ on molecule $i$. $a_{eff}$ is the size of a thermodynamically independent segment, which typically is in the range of 7 Å$^2$.

If $N_i = Nx_i$ is the number of molecules of species $i$ in the system, then the total number, $n$, of segments in the system is given by

$$n = \sum_i N_i n_i \tag{2}$$

In the same way, the number of segments of type $v$ is given by

$$n^v = \sum_i N_i n_i^v \tag{3}$$

The relative number or surface fraction of segments of type v is

$$\Theta^v = \frac{n^v}{n} \tag{4}$$

From elementary statistical thermodynamics we know that the Gibbs free energy of the system is

$$G = -k_B T \ln Z, \tag{5}$$

wherein Z is the total partition function of this system. The chemical potential of species $i$ in the mixture is given by

$$\mu_i = \frac{\partial G}{\partial N_i} = -k_B T \frac{\partial \ln Z}{\partial N_i}. \tag{6}$$

In view of an earlier assumption that the partition function $Z$ depends only on the segment composition it is

$$\mu_i = -k_B T \frac{\partial \ln Z}{\partial N_i} = -k_B T \sum_v \frac{\partial \ln Z}{\partial n^v} \frac{\partial n^v}{\partial N_i} = -k_B T \sum_v \frac{\partial \ln Z}{\partial n^v} n_i^v = \sum_v n_i^v \mu^v, \tag{7}$$

wherein $\mu^v$ is the pseudo-chemical potential of a segment of type $v$ in the ensemble of interacting surface segments. The activity coefficient, $\gamma_i$, of species $i$ is defined by

$$k_B T \ln \gamma_i = \mu_i - \mu_{ii} \qquad , \tag{8}$$

wherein $\mu_{ii}$ denotes the chemical potential of compound $i$ in a system with $x_i = 1$. In the same way it is

$$k_B T \ln \gamma^v = \mu^v - \mu^{vv} \quad , \tag{9}$$

for the activity coefficient of a segment of kind $v$, with $\mu^{vv}$ being the segment chemical potential at $\Theta^v = 1$. If $\mu_i^v$ and $\gamma_i^v$ is defined to be the chemical potential and activity coefficient of a segment $v$ in an ensemble of pure compound $i$, respectively, and these definitions is applied to equation 7, it is obtained

$$\ln \gamma_i = \frac{\mu_i - \mu_{ii}}{k_B T} = \sum_v n_i^v \frac{\mu^v - \mu_i^v}{k_B T}$$
$$= \sum_v n_i^v \frac{(\mu^v - \mu^{vv}) - (\mu_i^v - \mu^{vv})}{k_B T} = \sum_v n_i^v (\ln \gamma^v - \ln \gamma_i^v) \tag{10}$$

for the activity coefficient of compound $i$. By equation 10 the thermodynamics of the system of chemical compounds to the thermodynamics of an ensemble of pair-wise interacting surface segments are reduced.

Let $\mu$ and $v$ denote different kinds of surface segment and let

$$g_{\mu v} = h_{\mu v} - T s_{\mu v} \tag{11}$$

be the interaction energy of a pair $\mu v$. $g_{\mu v}$ in general may be a free energy of interaction of the two pairs, i.e. it may include enthalpic contributions $h_{\mu v}$ and entropic contributions $-T s_{\mu v}$, while mostly in literature only enthalpic interactions are considered. By the present invention it is assumed that symmetric interactions of the segments, i.e. $g_{\mu v} = g_{v \mu}$. The Boltzmann factor for the interaction energy of the segments is usually introduced as

$$\tau_{\mu v} = \exp\left\{ -\frac{g_{\mu v}}{k_B T} \right\} \tag{12}$$

In the COSMOSPACE paper [5] it is shown that a general and very efficient solution for the complete thermodynamics of such system can be achieved by solving the set of equation

$$\frac{1}{\gamma^v} = \sum_\mu \tau_{\mu v} \Theta^\mu \gamma^\mu \tag{13}$$

for the activity coefficients of segment types $v$ in the ensemble. In general this has to be solved numerically by iteration, usually starting from $\gamma^\mu = 1$ on the right hand side. In some cases, especially if only two different segment types are considered, analytic solutions can be derived. The efficiency of the COSMOSPACE equations results from the fact that via equation 10 the segment activity coefficients do directly lead to the activity coefficients and thus to the free energies of the compounds. The contact probabilities of segments are given by

$$p_{\mu v} = \Theta^\mu \gamma^\mu \tau_{\mu v} \gamma^v \Theta^v \equiv \Theta^\mu \Psi_{\mu v} \Theta^v \tag{14}$$

The enthalpy of the system is then given as expectation value of the interaction enthalpy, i.e.

$$H = \tfrac{1}{2} \sum_{\mu v} h_{\mu v} p_{\mu v}$$

As proven in appendix B of the COSMOSPACE paper [5], Gibbs-Duhem consistency of the resulting activity coefficients is automatically guaranteed within COSMOSPACE. Nevertheless in the derivation of the COSMOSPACE equations it was implicitly assumed that the segment interaction energies do not depend on the composition of the liquid system. But for mole fraction dependent segment interaction energies $g_{\mu v}(x)$ the activity coefficients of the compounds calculated by COSMOSPACE (equation10) are no longer Gibbs-Duhem consistent and hence thermodynamically wrong. This results from the fact that the additional composition dependence of the total free energy, which goes beyond the composition dependence resulting from the contact probabilities, and which is caused by the composition dependence of the interactions, is not taken into account in the variational solution of the statistical thermodynamics. Nevertheless, at a fixed value of x, the contact probabilities calculated within the COSMOSPACE framework must still be correct, because at a fixed value of x with the present invention interaction energies are advantageously fixed and hence no deviation from the assumptions made in COSMOSPACE.

Preferably an exact correction for the originally thermodynamic inconsistent activity coefficients of COSMOSPACE in the presence of composition dependent interaction energies can be derived, if we write the total free energy of a quasi-chemical system in the fundamental way

$$G = \frac{N}{2} \sum_{\mu v} \Theta^{\mu} \Theta^{v} \Psi_{\mu v} \left[ g_{\mu v} + \ln \Psi_{\mu v} \right] \qquad (15)$$

as described within the GEQUAC model [9].

Applying the definition of the compound chemical potentials according to equation 6

$$\mu_i = \frac{\partial G}{\partial N_i} = \frac{\partial}{\partial N_i} \left( \frac{N}{2} \sum_{\mu v} \Theta^{\mu} \Theta^{v} \Psi_{\mu v} \left[ g_{\mu v}(x) + \ln \Psi_{\mu v} \right] \right)$$
$$= \mu_i^0 + \frac{N}{2} \sum_{\mu v} \Theta^{\mu} \Theta^{v} \Psi_{\mu v} \left[ \frac{\partial g_{\mu v}(x)}{\partial N_i} \right] = \mu_i^0 + \frac{N}{2} \sum_{\mu v} p_{\mu v} \left[ \frac{\partial g_{\mu v}(x)}{\partial N_i} \right] \equiv \mu_i^0 + \mu_i^{corr} \qquad (16)$$

wherein the contribution of G was seperated, which arises from the composition dependence of the interactions, into $\mu_i^{corr}$ and subsumed all other parts into $\mu_i^0$. Therefore $\mu_i^0$ can preferably be calculated as usual from COSMO-SPACE , and $\mu_i^{corr}$ can preferably be calculated as the expectation value of the derivatives of the interaction energies with respect to the particle number. According to the invention equation 16 preferably is by itself a considerable extension of COSMOPACE models, because it allows for taking into account general composition dependences of the segment interactions, as long as the derivatives of the segment interaction energies on the composition can be calculated advantageously.

The euqations above provides a framework for working out the 2$^{nd}$ order COSMO-RS theory as follows:

It is assumed that segments $\mu$ and v have direct neighbor segments $\mu$' and v'. Due to the neighborhood of the segments a contact between $\mu$' and v' induces an increased local concentration of v in the environment of $\mu$ and vice versa, leading to an increased contact probability of $\mu$ and v. Any effects and energy corrections resulting from such neighbor induced local concentration are not taken into account in COSMO-RS and COSMOSPACE, nor in any quasi-chemical model so far. If the segments on the molecular surface would be in a planar region and of quadratic shape, then geometrically ¼ of the ($\mu$'v')-contacts would lead to a ($\mu$v)-contact. As in a model of a regular hexagonal lattice, the geometric correlation coefficient would reduce to 1/6. In the realistic case of segments on molecular surfaces, which have non-vanishing and irregular curvature, an exact evaluation of the induced local concentration of v in the surrounding of $\mu$ due to contacts of neighbor segments $\mu$' and v' will not be possible. If each segment preferably is counted on each compound as a separate type of segments, approximate expressions of the form

$$\widetilde{\Theta}_{\mu}^{v} = \sum_{\substack{\mu' \in i(\mu) \\ \mu' \neq \mu}} \sum_{\substack{v' \in j(v) \\ v' \neq v}} \Theta^{v'} \gamma^{v'} \tau_{\mu'v'} \gamma^{\mu'} f_{\mu'v'}^{corr} \qquad (17)$$

can be constructed for the induced local concentration of type v in the vicinity of segment type $\mu$, in which $i(\mu)$ and $j(v)$ denote the molecules $i$ and $j$ to which segments $\mu$ and v belong. The summations are over all 'neighbor' segments $\mu$ and v' of $\mu$ and v, respectively, i.e. over all segments on the same molecules. According to COSMOSPACE, $\Theta^{v'} \gamma^{v'} \tau_{\mu'v'} \gamma^{\mu}$ is the

probability that segment v' is partner of $\mu'$. The local correlation function $f_{\mu'v}^{corr}$ should decay rapidly with the distances $d(\mu'\mu)$ and $d(v'v)$ of the segments on the two molecules, as well as with the angles $\alpha(\mu'\mu)$ and $\alpha(v'v)$ between the normal vectors of the segment. Furthermore, it should be a kind of δ-function of the two distances, because only neighbor segments of $\mu$ and v at about the same distance can cause additional ($\mu$v) contacts. Thus a plausible functional form for $f_{\mu'v}^{corr}$ may preferably be

$$f_{\mu'v'}^{corr} \cong f_{corr}^0 \exp\left\{-\frac{d(\mu'\mu)^2}{d_{corr}^2}\right\} \exp\left\{-\frac{d(v'v)^2}{d_{corr}^2}\right\}$$

$$* \exp\left\{-\frac{\alpha(\mu'\mu)^2}{\alpha_{corr}^2}\right\} \exp\left\{-\frac{\alpha(v'v)^2}{\alpha_{corr}^2}\right\} \exp\left\{-\frac{|d(\mu'\mu)-d(v'v)|^2}{d_{tol}^2}\right\}$$

(18)

but the detailed and optimal expression for the correlation function is not relevant for the purpose of the present invention. It will have to be worked out based on plausible assumptions and fitting of a few empirical parameters. The relevant fact is that the induced local concentration $\widetilde{\Theta}_\mu^v$ of v in the surrounding of $\mu$ is given by a sum of neighbor contact probabilities, which are known from the initial COSMOSPACE calculation, and a geometric function, which can be easily calculated from the segment coordinates stored in the COSMO files. Indeed, the intramolecular segment distances and normal vector angles can be precalculated and stored in each COSMO file, making the later evaluation of the correlation functions more efficient. If, as usual in COSMO-RS, several segments, e.g. segments of similar polarization charge densities σ, are comprised to one segment type, the appropriate summations and averages of the induced local concentrations have to be calculated. But still the induced contact probabilities will be summations of COSMOSPACE contact probabilities multiplied by geometric correlation functions.

If the sum of all induced local concentrations at $\mu$ preferably is introduced as

$$\widetilde{\Theta}_\mu^{tot} = \sum_v \widetilde{\Theta}_\mu^v$$

(19)

it can be defined a locally corrected concentration of v in the vicinity of $\mu$ as

$$\hat{\Theta}_\mu^v = \frac{\Theta^v + \widetilde{\Theta}_\mu^v}{1 + \widetilde{\Theta}_\mu^{tot}}$$

,

(20)

assuming that the global concentration $\Theta^v$ of segment type v adds to the induced local concentrations. The denominator in equation 20 results from normalization. Thus the ratio of the locally corrected concentration and the initially used global concentration of v in the vicinity of $\mu$ is given by the ratio of $\hat{\Theta}_\mu^v$ and $\Theta^v$. If this is converted into an entropic correction for the contact probability of $\mu$ and v, it is obtained

$$g_{\mu v}^{lcc} = -k_B T f_{corr}\left[\ln\left\{\frac{(1-\widetilde{\Theta}_\mu^{tot})\Theta^v + \widetilde{\Theta}_\mu^v}{\Theta^v}\right\} + \ln\left\{\frac{(1-\widetilde{\Theta}_v^{tot})\Theta^\mu + \widetilde{\Theta}_v^\mu}{\Theta^\mu}\right\}\right]$$

,

(21)

wherein $f_{corr}$ is a factor.

Preferably the factor $f_{corr}$ is ½ avoiding double counting, because both induced concentrations will be preferably simultaneously corrected in order to get a symmetric free energy correction for the contacts of $\mu$ and v, resulting in calculating as follows:

$$g_{\mu v}^{lcc} = -\frac{k_B T}{2}\left[\ln\left\{\frac{\Theta^v + \widetilde{\Theta}_\mu^v}{(1+\widetilde{\Theta}_\mu^{tot})\Theta^v}\right\} + \ln\left\{\frac{\Theta^\mu + \widetilde{\Theta}_v^\mu}{(1+\widetilde{\Theta}_v^{tot})\Theta^\mu}\right\}\right]$$

(21.1)

Now 2nd order interaction free energies can be introduced

$$g_{\mu\nu}^{*} = g_{\mu\nu} + g_{\mu\nu}^{lcc} \tag{22}$$

and solve the COSMOSPACE equations again, yielding 2nd order segment activity coefficients $\gamma_{*}^{\mu}$ and new contact probabilities $p_{\mu\nu}^{*}$ as well as a new total enthalpy, which will reflect the geometry induced local correlation of segment types. Since the $g_{\mu\nu}^{lcc}$ include the first order contact probabilities and by that depend on the composition of the system, the calculation of free energies and activity coefficients of the compounds in the system requires the correction term as given in equation 16. Hence the free energies of compound $i$ in the system are as calculated as follows:

$$\mu_{i}^{*} = \mu_{i}^{0} + \mu_{i}^{*corr} = k_{B}T\sum_{\nu\in i}\ln\gamma_{*}^{\nu} + \frac{N}{2}\sum_{\mu\nu}p_{\mu\nu}^{*}\left[\frac{\partial g_{\mu\nu}^{lcc}}{\partial N_{i}}\right] \tag{23}$$

The $g_{\mu\nu}^{lcc}$ depend on the composition and by that on the particle number $N_{i}$ only via the surface fractions and 1st order contact probabilities, and thus on the 1st order segment activity coefficients $\gamma^{\mu}$. The analytic derivatives of the latter with respect to the particle numbers can be calculated, as described in detail in appendix D of the COSMOSPACE paper.

Thus according to the invention, preferably according to the inventive simulation/calculating method above, the complete 2nd order fluid phase thermodynamics of the system is accomplished, which recovers local correlations of the surface segments on the molecular surfaces, which was ignored in the 1st order COSMO-RS and COSMOSPACE equations, as well as in any kind of quasi-chemical approach.

EXAMPLE

For the validation of the COSMO-RSC theory it is useful to consider a case in which the correlation effects can be clearly identified and quantified, and which is simple enough in order to construct the surface correlation function analytically. Such rather realistic model systems can be provided by lattice fluids and lattice Monte-Carlo simulations. Such systems have already been considered during the original development of COSMO-RS, and later such LMC calculations with cubic pseudo-molecules have been used for validation purposes in the COSMOSPACE paper.

In order to study substantial neighbor correlation effects the cubic molecules are not sufficient. Therefore it will be considered in the following a lattice fluid consisting of $N_{c}$ cubic pseudo-molecules, and $N_{t}$ straight triples molecules, which are just 3 cubes stuck together linearly. Thus the cube molecules have 6 surface segments, and the triples have 14 external surface segments. In general each of the 20 segments may be of different type v. A lattice Monte-Carlo program triple-LMC was written, which allows the specification of a polarization charge density $\sigma_{\nu}$ on each segment type v, and the interaction energies are given by the generic functional form

$$g_{\mu\nu}^{LMC} = h_{\mu\nu}^{LMC} = \sigma_{\nu}\sigma_{\mu} \tag{24}$$

The present invention prefearbly considers a cubic simulation box of size $L^{3}$ (default $L = 20$) and apply periodic boundary condition. Since it will be considered a densely filled box, the number of cube molecules is given by

$$N_{c} = L^{3} - 3N_{t} \tag{25}$$

The four types of LMC moves required in such system are quite simple:

1) random rotation of a cube into one of its 24 possible orientations.
2) random rotation by 90° or 180° of a triple either around its long axis or rotation by 180° around one of the perpendicular axes.
3) selection of a triple which has a cube at one of its head faces and exchange of the two molecules.
4) selection of a triple and of three linearly neighboring cubes and exchange of the triple with and the three cubes.

These LMC moves guarantee that all arrangements of the LMC mixture can be achieved. It is performed $2*10^7$ LMC steps for each composition and temperature and used the last half of the LMC steps for the evaluation of the average interaction enthalpy $H_{LMC}$. It is made no attempts to derive free energies and activity coefficients from the LMC simulations, because that would be much more complicated than the evaluation of the interaction enthalpy. By the proper choice of polar and less polar surface segments, i.e. by the proper choice of the 20 types of polarization charge densities $\sigma_v$ on the faces of the cube and triple molecules, many different systems can be mimicked, in which different types of neighbor correlation effects can be studied.

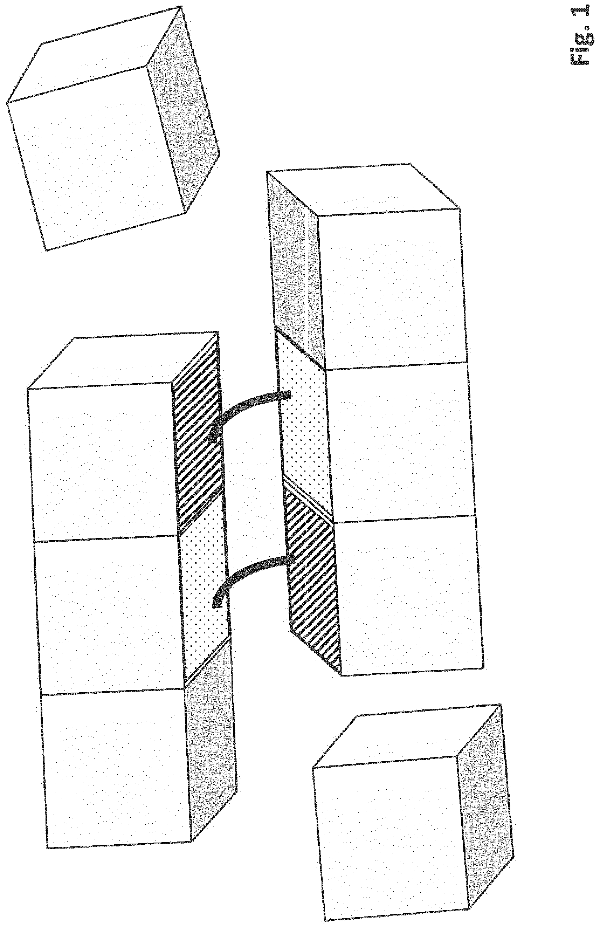

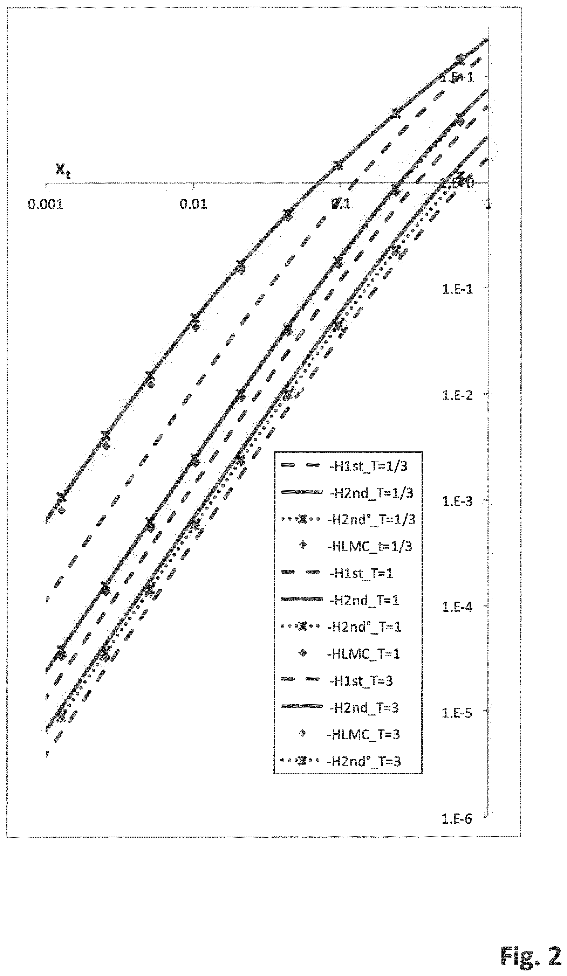

For the analytic validation of COSMO-RSC (shown in the figures) a very simple but quite realistic case is preferably be considered , mimicking a mixture of methane and acetic acid, where the pseudo-methane is represented by the cubes, with all surface segments being neutral (or green, if the standard color coding of COSMO-RS is used), and the acetic acid molecules are represented by triple molecules, which have a strongly negatively polar surface segment representing a hydrogen bond acceptor (i.e. a dot pattern segment with positive $\sigma$) in the middle of one of the long sides, and a positively polar segment representing a hydrogen bond donor (i.e. a stripe pattern segment with negative $\sigma$) directly adjacent to the dot pattern acceptor segment. The cubic and triple molecules are schematically visualized in figure 1. LMC simulation have been performed for this cube-triple mixture over a wide range of compositions expressed as mole fractions of triples $x_t$ and temperatures, expressed in units of $\sigma^2/k_B$, where $\sigma$ is the polarization charge density of the positively polar segment on the triple. The resulting interaction enthalpies $H_{LMC}(x_t,T)$ are shown in figure 2.

Next the 2nd order COSMO-RS calculation can be performed for this system. Therefore, the 1st order COSMO-SPACE equations has to be solved firstly. To simplify the calculation all neutral (non pattern), non-polar surface segments are denotes as type A, and the donor and acceptor segments are denoted as types B and B'. Since the polarities of the donor and acceptor segments are symmetric, and since their surface fractions are always identical, their segment activity coefficients $\gamma^B$ and $\gamma^{B'}$ must be identical. Because the neutral segments (non pattern) have $\sigma_A = 0$, the Boltzmann factors $\tau_{AA}$, $\tau_{AB}$ and $\tau_{AB'} =1$ are unity. Denoting the Boltzmann factor of a BB' contact as $\tau = \tau_{BB'}$, the Boltzmann factors for the like contacts are $\tau_{BB}=\tau_{B'B'}=1\tau$. Then the COSMOSPACE equations become:

$$1 = \Theta^A \gamma^{A^2} + \Theta^B \gamma^B \gamma^A + \Theta^{B'} \gamma^{B'} \gamma^A = \Theta^A \gamma^{A^2} + 2\Theta^B \gamma^B \gamma^A \qquad (26)$$

and

$$1 = \Theta^{B'} \gamma^{B'} \gamma^B \tau^{-1} + \Theta^B \gamma^{B^2} \tau + \Theta^A \gamma^A \gamma^B = \Theta^B \gamma^{B^2} \tau^{-1} + \Theta^B \gamma^{B^2} \tau + \Theta^A \gamma^A \gamma^B \qquad (27)$$

with $\theta^B = \theta^B = (1-\theta^A)/2$. Solving eq. 26 for $\gamma^B$ and inserting this into eq. 27 leads to a quadratic equation with respect to $\gamma^{A^2}$ which can be solved by standard algebraic methods.

Thus the segment activity coefficients is yielded and the enthalpy $H_{CRS1}(x_t,T)$ of the system according to standard, 1st order COSMO-RS is calculated. As can be seen in figure 2, these first order results deviate substantially from the LMC results. The reason is the strong local correlation of the neighboring donor and acceptor segments, B and B'.

While the geometry of the triple molecules also induces some correlation between A type segments (neutral, non pattern), this will be very small, because type A segments are plentiful at all compositions of the system. The induced correlation of BB contact due to B'B' contacts can also be neglected, since like contacts of the polar segments have an extremely low probability. Therefore the effects of the correlation between B and B' due to neighboring B' and B contacts will clearly be dominating. For the sake of clarity and simplicity it will be preferably only considered the induced local concentration of this type. According to eq. 17, the induced local concentration of B' in the vicinity of B is

$$\widetilde{\Theta}^{B'}_B = \tfrac{1}{4}\Theta^B \gamma^B \tau \gamma^{B'} = \tfrac{1}{4}\Theta^B \gamma^{B^2} \tau \qquad (28)$$

Using equations 20 and 21.1 the local concentrations free energy corrections are yielded

$$g^{lcc}_{BB'} = -\frac{k_B T}{2}\left[ \ln\left\{ \frac{\Theta^{B'} + \widetilde{\Theta}^{B'}_B}{(1+\widetilde{\Theta}^{B'}_B)\Theta^{B'}} \right\} + \ln\left\{ \frac{\Theta^B + \widetilde{\Theta}^B_{B'}}{(1+\widetilde{\Theta}^B_{B'})\Theta^B} \right\} \right]$$

$$= -k_B T\left[ \ln\{\Theta^B + \widetilde{\Theta}^{B'}_B\} - \ln\{1+\widetilde{\Theta}^{B'}_B\} - \ln\{\Theta^B\} \right] \equiv -k_B T[\ln\{s\}] \qquad (29)$$

$$g_{BA}^{lcc} = -\frac{k_B T}{2}\left[\ln\left\{\frac{\Theta^A}{\left(1+\widetilde{\Theta}_B^{B'}\right)\Theta^A}\right\}\right] = \frac{k_B T}{2}\ln\left\{1+\widetilde{\Theta}_B^{B'}\right\} \equiv -k_B T\ln\{r\}$$ (30)

and

$$g_{BB}^{lcc} = -\frac{k_B T}{2}\left[\ln\left\{\frac{\Theta^B}{\left(1+\widetilde{\Theta}_B^{B'}\right)\Theta^B}\right\}+\ln\left\{\frac{\Theta^{B'}}{\left(1+\widetilde{\Theta}_{B'}^{B}\right)\Theta^{B'}}\right\}\right] = k_B T\ln\left\{1+\widetilde{\Theta}_B^{B'}\right\} = -k_B T\ln\left\{r^2\right\}$$ (31)

Using the shortcuts s and r defined in equations 29 and 30, the COSMOSPACE equations for the 2nd order COSMO-RS correction become:

$$1 = \Theta^A\gamma_*^{A^2} + 2r\Theta^B\gamma_*^B\gamma_*^A$$ (32)

$$1 = \Theta^B\gamma_*^{B^2}r^2\tau^{-1} + \Theta^B\gamma_*^{B^2}s\tau + \Theta^A r\gamma_*^A\gamma_*^B$$ (33)

The segment activity coefficients $\gamma_*^A$ and $\gamma_*^B$ can now again be calculated analytically from eqs. 32 and 33 by standard algebra. From this the 2nd order enthalpy $H_{CRS2}(x_t,T)$ can be calculated. This is shown in figure 2 together with the LMC enthalpy and the 1st order COSMO-RS result. As can be seen, $H_{CRS2}(x_t,T)$ is in good agreement with the LMC results. Especially at low temperatures, where correlation effects are most important deviations between 1st order COSMO-RS are largest, the 2nd order COSMO-RS results agree very well with the LMC simulations. At higher temperatures the LMC simulations are almost half way between the 1st and 2nd order COSMO-RS results and the agreement appears to be less good on a logarithmic scale, but on an absolute scale the deviations here are very small.

Preferably in figure 2 the results achieved by numerical COSMO-RSC calculations which take into account all correlations, and not just the correlation between the polar surface segments are added. These results agree perfectly with the analytical results at lower temperatures, while at high temperature (T = 3) these results slightly deviate from the simplified analytical model and given an even better description of the LMC results.

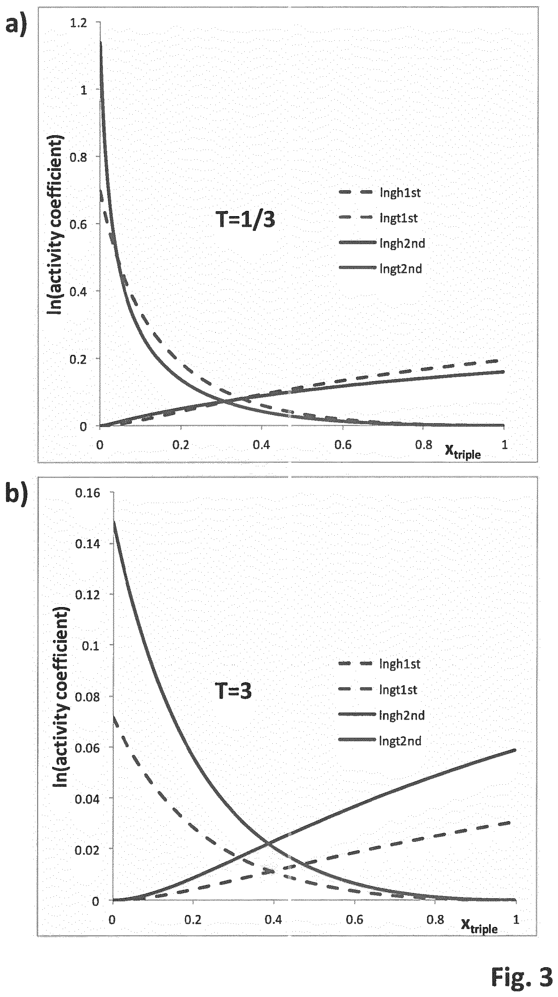

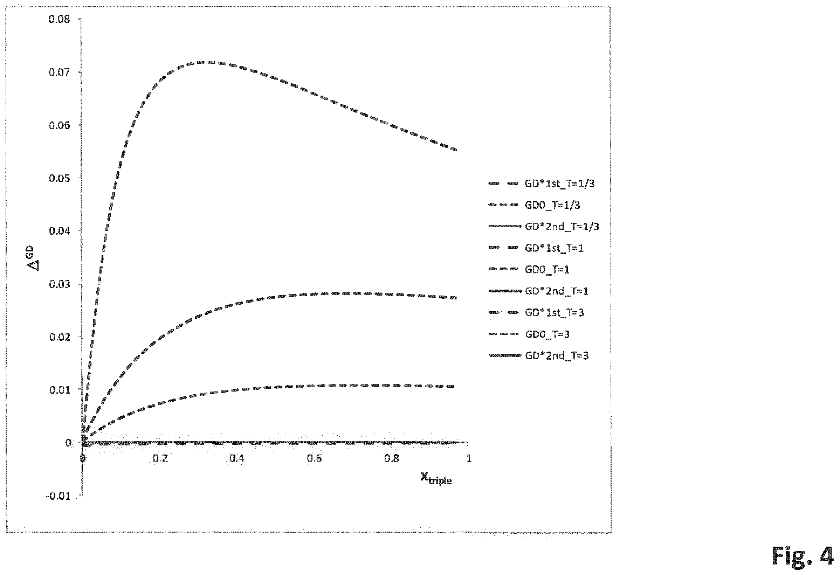

In a last calculation step of the present simulation method it needs to calculate the free energies $\mu_c$ and $\mu_t$ of the cube and triple molecules according to equation 23. The uncorrected contributions $\mu_c^0$ and $\mu_t^0$ can be derived by summing up the logarithmic 2nd order segment activity coefficients. Since there are explicit analytic expressions for the 1st order segment activity coefficients, the analytic derivatives of the $\gamma^A$ and $\gamma^B$ can prefearbly be calculated with respect to the surface fraction $\theta^A$ of non-polar segments. From that the derivatives of the entropic local correlation corrections are obtained. Combining these with the derivatives of the $\theta^A$ with respect to the particle numbers $N_c$ and $N_t$, respectively, the derivatives of the interaction energy corrections can be yielded with respect to the particle numbers. Following by combining these with the second order contact probabilities the desired chemical potential corrections $\mu_c^{*corr}$ and $\mu_t^{*corr}$ and the total chemical potentials of both species are obtained. Figure 3 shows the chemical potentials resulting from first order COSMO-RS and the uncorrected and corrected chemical potentials resulting from 2nd order COSMO-RS of the compounds at three different temperatures. Figure 4 shows the corresponding Gibbs-Duhem consistency tests

$$\Delta^{GD} = \frac{x_c}{k_B T}\frac{\partial\mu_c}{\partial x_c} + \frac{x_t}{k_B T}\frac{\partial\mu_t}{\partial x_c}$$

for all three types of chemical potentials displayed in figure 3. Consistency is given if $\Delta^{GD}$ is zero. The derivatives of the chemical potentials are calculated numerically. As can be seen in figure 4, the 1st order chemical potentials and the corrected 2nd order chemical potentials are perfectly Gibbs-Duhem consistent at all temperatures, while the uncorrected 2nd order chemical potential are massively inconsistent.

Thus, the present invention disclose a 2nd order surface polarity correlation correction, COSMO-RSC, which preferably makes use of the contact probabilities derived from standard 1st order COSMO-RS calculations, derives from these local concentrations of surface polarities and feeds them back as entropic interaction free energy corrections into a second COSMO-RS loop. Utilizing a novel, thermodynamically exact correction method, the resulting 2nd order free energies and activity coefficients are completely Gibbs-Duhem consistent. As shown on the example of a lattice Monte-Carlo fluid mimicking the dimerization of acetic acid in alkane solvents, the 2nd order COSMO-RS thermodynamics recovers the initially neglected effects resulting from coordinated interactions of neighboring surface segments almost perfectly.

In the model example considered in this invention the computational costs of COSMO-RSC are just about 3 times the costs of simple COSMO-RS, because the COSMOSPACE equations have to be solved a second time and derivatives of the contact probabilities have to be evaluated. In COSMO-RS calculations for real molecules most likely the evaluation of the geometrical segment correlation functions will be the major computational task in COSMO-RSC, because they involve the evaluation of many distances and angles between segments and segment normal vectors, respectively. Nevertheless, much of that can be precalculated and stored as an appendix to the COSMO file, which holds the polarity and geometric information of each molecule. Compared to the quantum chemical calculation required for the generation of the COSMO file the calculation of the segment correlation functions will be only a small additional effort. By such techniques it can be expected that the increase in computational costs caused by COSMO-RSC will be in the order of a factor of 3 or less.

Thus it can be expected, while keeping the thermodynamic consistency and much of the efficiency of the COSMO-RS approach, COSMO-RSC will lead to improved overall accuracy for the prediction of thermodynamic properties, because the so far neglected effects of surface polarity correlation will be taken into account to a large degree. Furthermore, many extension of the application range can be expected, e.g. aggregation phenomena, hydrotropes, micro phase separation or even hydrophobic collapse of long alkyl or polymer chains. The potential as well the limits are currently not foreseeable.

According to the present invention even higher order COSMO-RS, i.e. using the COSMO-RSC information for correlation effects in a 3rd COSMO-RS loop, can be performed, also, preferably additionally to the inventive simulation/calculating method. Since the composition derivatives of all quantities entering into the 2nd order COSMO-RS are available, the derivatives of the resulting contact probabilities should be available as well and thus even 3rd order and higher order COSMO-RS should yield thermodynamically consistent chemical potentials.

Another aspect of the invention relates to a computer apparatus according to claim 9 for simulation, preferably for calculation, of at least one physical property of a system of one or more chemical species that includes at least one chemical species dissolved in at least one chemical species, using a quasi-chemical, COSMO-RS, COSMO-SAC, or COSMOSPACE calculation involving composition dependent segment interactions $g_{\mu v}$ and achieving Gibbs-Duhem consistent chemical potentials and activitiy coefficients or Gibbs-Duhem consistent chemical potentials or Gibbs-Duhem consistent activitiy coefficients.

The claimed method is computer-implemented. programmed software can be stored on a storage medium as for example on a mobile storage medium, on a clients computer and/or on a server computer processing, storage, and input/output devices executing application programs and the like.

In a preferred embodiment of the invention the simulation can be visualized as a computerized model on a display device and/or the computerized model can be printed or plotted, for example as a 3D-model printed with a 3D-printer. Therefore, also disclosed is a computerized model for visualization of the simulation and/or the calculation.

In order to avoid repetition with respect to further advantages of the computer apparatus according to the invention, reference is made to the description of the advantageous refinement of the method of the invention and it will be fully resorted to this.

PREFERRED EMBODIMENT

Further, the invention will become apparent from the following description of an preferred embodiment of the invention, which is shown schematically in the figures.

The essential features of the invention aredefined in the appended claims. It should be noted that the figures are only descriptive and are not intended to limit the invention in any way. It shows in a schematic illustration:

Fig. 1    a computerized model cubic and triple molecules (cube-triple mixture) with adjacent oppositely polar surface segments,

Fig. 2    a diagram showing resulting interaction enthalpies $H_{LMC}(x_t,T)$ by performing LMC simulation for the cube-triple mixture of Figure 1,

Fig. 3    a diagram showing the chemical potentials resulting from first order COSMO-RS and the uncorrected and corrected chemical potentials resulting from 2nd order COSMO-RS of the compounds for the cubic and triple molecules of figure 1 at three different temperatures a) at T = 1/3, b) at T = 3 and

Fig. 4    a diagram showing Gibbs-Duhem consistency test for the 1st and 2nd order chemical potentials as well as for the raw 2nd order chemical potentials before correction for all three types of chemical potentials displayed in figure 3.

Figure 1 shows a schematically visualisation of cubic and triple molecules mimicking a mixture of methane and acetic acid, where the pseudo-methane is represented by the cubes, with all surface segments being neutral (or neutral, non pattern; typically standard color coding of COSMO-RS is green), and the acetic acid molecules are represented by triple molecules, which have a strongly negatively polar surface segment representing a hydrogen bond acceptor (i.e. a dot pattern segment with positive $\sigma$) in the middle of one of the long sides, and a positively polar segment representing a hydrogen bond donor (i.e. a stripe pattern segment with negative $\sigma$) directly adjacent to the dot pattern acceptor segment. Non-polar neutral non pattern cube molecules and triple molecules with adjacent oppositely polar surface segments. Due to their direct neighborhood the polar segments on the triples can form a very favorable double interaction.

Figure 2 shows in a diagram LMC simulation, that have been performed for the cube-triple mixture of figure 1 over a wide range of compositions expressed as mole fractions of triples xt and temperatures, expressed in units of $\sigma^2/k_B$, where $\sigma$ is the polarization charge density of the positively polar segment on the triple. The resulting interaction enthalpies $H_{LMC}$ $(x_t, T)$ are shown in the figure.

Figure 3 shows in a diagram 1st order and 2nd order COSMO-RS activity coefficients for the cube and triple molecules, wherein a) shows analytical results at higher temperature $T = 1/3$ and b) shows analytical results at lower temperature $T = 3$.

Figure 4 shows in a diagram Gibbs-Duhem consistency test for the 1st and 2nd order chemical potentials as well as for the raw 2nd order chemical potentials before correction. The latter show a massive deviation from Gibbs-Duhem consistency, while the 1st order and the corrected 2nd order chemical potentials are perfectly Gibbs-Duhem consistent at all temperatures.

**List of references cited**

[1] (1) Klamt, A. Conductor-like Screening Model for Real Solvents: A New Approach to the Quantitative Calculation of Solvation Phenomena. J. Phys. Chem. 1995, 99 (7), 2224-2235.

[2] Klamt, A.; Jonas, V.; Bürger, T.; Lohrenz, J. C. Refinement and Parametrization of COSMO-RS. J. Phys. Chem. A 1998, 102 (26), 5074-5085.

[3] Klamt, A. COSMO-RS From Quantum Chemistry to Fluid Phase Thermodynamics and Drug Design; Elsevier: Amsterdam, The Netherlands; Boston, MA, USA, 2005.

[4] Klamt, A.; Schüürmann, G. COSMO: A New Approach to Dielectric Screening in Solvents with Explicit Expressions for the Screening Energy and Its Gradient. J. Chem. Soc. Perkin Trans. 2 1993, 1993 (5), 799-805.

[5] Klamt, A.; Krooshof, G. J. P.; Taylor, R. COSMOSPACE: Alternative to Conventional Activity-Coefficient Models. AIChE J. 2002, 48 (10), 2332-2349.

[6] Klamt, A.; Eckert, F.; Arlt, W. COSMO-RS: An Alternative to Simulation for Calculating Thermodynamic Properties of Liquid Mixtures. Annu. Rev. Chem. Biomol. Eng. 2010, 1 (1), 101-122.

[7] Lucas, K. Molecular Models for Fluids; Cambridge University Press: Cambridge , NY, USA, 2007.

[8] Guggenheim, E. A.; Turgeon, J. C. Specific Interaction of Ions. Trans. Faraday Soc. 1955, 51, 747-761.

[9] Egner, K.; Gaube, J.; Pfennig, A. GEQUAC, an Excess Gibbs Energy Model Describing Associating and Nonassociating Liquid Mixtures by a New Model Concept for Functional Groups. Fluid Phase Equilibria 1999, 158, 381-389.

[10] Abrams, D. S.; Prausnitz, J. M. Statistical Thermodynamics of Liquid Mixtures: A New Expression for the Excess Gibbs Energy of Partly or Completely Miscible Systems. AIChE J. 1975, 21 (1), 116-128.

[11] Fredenslund, A.; Jones, R. L.; Prausnitz, J. M. Group-Contribution Estimation of Activity Coefficients in Nonideal Liquid Mixtures. AIChE J. 1975, 21 (6), 1086-1099.

[12] Larsen, B. L.; Rasmussen, P. A Comparison between the Quasichemical Model and Two-Fluid Local-Composition Models. Fluid Phase Equilibria 1986, 28 (1), 1-11.

[13] Lin, S.-T.; Sandler, S. I. Reply to Comments on "A Priori Phase Equilibrium Prediction from a Segment Contribution Solvation Model." Ind. Eng. Chem. Res. 2002, 41 (9), 2332-2334.

[14] Sachsenhauser, T.; Rehfeldt, S.; Klamt, A.; Eckert, F.; Klein, H. Consideration of Dimerization for Property Prediction with COSMO-RS-DARE. Fluid Phase Equilibria 2014, 382, 89-99.

**Claims**

1. A method for selecting solvents for a reaction step using a computer-implemented method for simulation of at least one physical property of a system of one or more chemical species that includes at least one chemical species dissolved in at least one chemical species, using a second quasi-chemical, COSMO-RS, COSMO-SAC, or COSMOSPACE calculation involving composition dependent segment interactions $g_{\mu v}$ and achieving Gibbs-Duhem consistent chemical potentials and activity coefficients or Gibbs-Duhem consistent chemical potentials or Gibbs-Duhem consistent activity coefficients, **characterized in that**
   segment contacting probabilities, resulting from a first quasi-chemical, COSMO-RS, COSMO-SAC or COSMOSPACE calculation for quantifying the induced local concentrations of surface types or polarization charge densities due to the neighbor segments of contacting neighbor segments, are used as an input variable in the second calculation, the second calculation thereby resulting in modified segment contact probabilities which reflect the local correlations of surface polarities, wherein the induced local concentrations are converted into an entropic correction for the pair-wise interaction of segments and the second calculation uses the local concentration corrected interaction energies.

2. The method according to claim 1, comprising wherein the method for simulation comprises computing the chemical potential $\mu_i$ of each species i by computing the following equation:

$$\mu_i^* = \mu_i^0 + \mu_i^{*corr} = k_B T \sum_{v \in i} \ln \gamma_*^v + \frac{N}{2} \sum_{\mu v} p_{\mu v}^* \left[ \frac{\partial g_{\mu v}}{\partial N_i} \right],$$

wherein:

   $k_B$ is the Boltzmann constant;
   T is the temperature;
   $v \in i$ designates the set of segment types $v$ on species $i$;

   $\gamma_*^v$ is the uncorrected segment activity coefficient of the segment type $v$;
   $N$ is the number of molecules in the system;
   $N_i$ is the number of molecules of species $i$;
   $\mu v$ designates a pair of segment types $\mu$ and $v$;
   $g_{\mu v}$ is the interaction energy for the pair $\mu v$; and

   $p_{\mu v}^*$ is the segment contacting probability for segment types $\mu$ and $v$.

3. The method according to any one of the preceding claims, **characterized in that** at least one increased local concentration of one or more neighbor segments of at least one molecule in the environment of at least one neighbor segment of at least one other molecule is corrected in form of an entropic interaction, wherein the at least two molecules may be of the same or of different chemical species.

4. The method according to any one of the preceding claims includes calculating:

$$g_{\mu v}^{lcc} = -k_B T f_{corr} \left[ \ln \left\{ \frac{(1 - \tilde{\Theta}_\mu^{tot}) \Theta^v + \tilde{\Theta}_\mu^v}{\Theta^v} \right\} + \ln \left\{ \frac{(1 - \tilde{\Theta}_v^{tot}) \Theta^\mu + \tilde{\Theta}_v^\mu}{\Theta^\mu} \right\} \right],$$

wherein:

$k_B$ is the Boltzmann constant;

T is the temperature;

$\Theta^v$ is the surface fraction of segment type $v$;

$\widetilde{\Theta}_\mu^v$ is an approximation of the induced concentration of type segment $v$ in the vicinity of segment type $\mu$;

$\widetilde{\Theta}_\mu^{tot} = \sum_v \widetilde{\Theta}_\mu^v$ is the sum of all induced concentrations at segment type $\mu$; and

$f_{corr}$ is a factor, in particular wherein the factor $f_{corr}$ is ½ avoiding double counting, in particular resulting in calculating as follows:

$$g_{\mu v}^{lcc} = -\frac{k_B T}{2}\left[\ln\left\{\frac{\Theta^v + \widetilde{\Theta}_\mu^v}{\left(1 + \widetilde{\Theta}_\mu^{tot}\right)\Theta^v}\right\} + \ln\left\{\frac{\Theta^\mu + \widetilde{\Theta}_v^\mu}{\left(1 + \widetilde{\Theta}_v^{tot}\right)\Theta^\mu}\right\}\right]$$

5. The method according to any one of the preceding claims, **characterized in that** free energy of at least one chemical species in the system is calculated.

6. The method according to any one of the preceding claims, **characterized in that** chemical potential with respect to the local concentration interaction of the at least one neighbor segment of the one or more chemical species in the environment of the at least one neighbor segment of the same or another chemical species is corrected.

7. The method according to any one of the preceding claims, in particular according to claim 5, including calculating the chemical potential $\mu_i$ of each species $i$ by calculating:

$$\mu_i = \frac{\partial G}{\partial N_i} = \frac{\partial}{\partial N_i}\left(\frac{N}{2}\sum_{\mu v}\Theta^\mu\Theta^v\Psi_{\mu v}\left[g_{\mu v}(x) + \ln\Psi_{\mu v}\right]\right)$$

$$= \mu_i^0 + \frac{N}{2}\sum_{\mu v}\Theta^\mu\Theta^v\Psi_{\mu v}\left[\frac{\partial g_{\mu v}(x)}{\partial N_i}\right] = \mu_i^0 + \frac{N}{2}\sum_{\mu v}p_{\mu v}\left[\frac{\partial g_{\mu v}(x)}{\partial N_i}\right] \equiv \mu_i^0 + \mu_i^{corr}$$

wherein:

$G$ is the total free energy of the system, with $G = \frac{N}{2}\sum_{\mu v}\Theta^v\Theta^\mu\Psi_{\mu v}\left[g_{\mu v} + \ln\Psi_{\mu v}\right]$

$N_i$ is the number of molecules of species $i$;

$N$ is the number of molecules in the system;

$\Theta^v$ is the surface fraction of segment type $v$;

$\mu v$ designates a pair of segment types $\mu$ and $v$;

$p_{\mu v}$ is the segment contacting probability for the pair $\mu v$, with $p_{\mu v} = \Theta^\mu\Psi_{\mu v}\Theta^v$; and

$g_{\mu v}$ is the interaction energy for the pair $\mu v$.

8. The method according to any one of the preceding claims, **characterized in that** at least one of the calculation steps will be repeated at least in once, preferably in a 2nd loop or higher.

9. A computer apparatus for simulation of at least one physical property of a system of one or more chemical species that includes at least one chemical species dissolved in at least one chemical species, using a quasi-chemical, COSMO-RS, COSMO-SAC, or COSMOSPACE calculation involving composition dependent segment interactions $g_{\mu v}$ and achieving Gibbs-Duhem consistent chemical potentials and activity coefficients or Gibbs-Duhem consistent chemical potentials or Gibbs-Duhem consistent activity coefficients, wherein the computer apparatus is configured to carry out the method of at least one of the preceding claims 1 to 8.

**Patentansprüche**

1. Verfahren zum Auswählen von Lösungsmitteln für einen Reaktionsschritt unter Verwendung eines rechnerimplementierten Verfahrens zur Simulation von mindestens einer physikalischen Eigenschaft eines Systems aus einer oder mehreren chemischen Spezies, das mindestens eine chemische Spezies beinhaltet, die in mindestens einer chemischen Spezies gelöst ist, unter Verwendung einer zweiten quasi-chemischen, COSMO-RS-, COSMO-SAC- oder COSMOSPACE-Berechnung, die zusammensetzungsabhängige Segmentwechselwirkungen $g_{\mu v}$ involviert und mit Gibbs-Duhem konsistente chemische Potentiale und Aktivitätskoeffizienten oder mit Gibbs-Duhem konsistente chemische Potentiale oder mit Gibbs-Duhem konsistente Aktivitätskoeffizienten erreicht, **dadurch gekennzeichnet, dass** Segmentberührungswahrscheinlichkeiten, die aus einer ersten quasi-chemischen, COSMO-RS-, COSMO-SAC- oder COSMOSPACE-Berechnung zum Quantifizieren der induzierten lokalen Konzentrationen von Oberflächentypen oder Polarisationsladungsdichten aufgrund der Nachbarsegmente von sich berührenden Nachbarsegmenten resultieren, bei der zweiten Berechnung als Eingangsgröße verwendet werden,
   wodurch die zweite Berechnung in modifizierten Segmentberührungswahrscheinlichkeiten resultiert, die die lokalen Korrelationen von Oberflächenpolaritäten widerspiegeln, wobei die induzierten lokalen Konzentrationen in eine entropische Korrektur für die paarweise Wechselwirkung von Segmenten umgewandelt werden und die zweite Berechnung die durch die lokale Konzentration korrigierten Wechselwirkungsenergien verwendet.

2. Verfahren nach Anspruch 1, umfassend wobei das Simulationsverfahren ein Berechnen des chemischen Potenzials $\mu_i$ jeder Spezies i durch Berechnen der folgenden Gleichung umfasst:

$$\mu_i^* = \mu_i^0 + \mu_i^{*corr} = k_B T \sum_{v \varepsilon i} \ln \gamma_*^v + \frac{N}{2} \sum_{\mu v} p_{\mu v}^* \left[ \frac{\partial g_{\mu v}}{\partial N_i} \right],$$

   wobei:

   > $k_B$ die Boltzmann-Konstante ist;
   > T die Temperatur ist;
   > $v \varepsilon i$ den Satz von Segmenttypen $v$ an Spezies $i$ bezeichnet;
   > $\gamma_*^v$ der unkorrigierte Segmentaktivitätskoeffizient des Segmenttyps $v$ ist;
   > N die Anzahl an Molekülen in dem System ist;
   > $N_i$ die Anzahl an Molekülen von Spezies $i$ ist;
   > $\mu v$ ein Paar von Segmenttypen $\mu$ und $v$ bezeichnet;
   > $g_{\mu v}$ die Wechselwirkungsenergie für das Paar $\mu v$ ist; und
   >
   > $p_{\mu v}^*$ die Segmentberührungswahrscheinlichkeit für Segmenttypen $\mu$ und $v$ ist.

3. Verfahren nach einem der vorherigen Ansprüche, **dadurch gekennzeichnet, dass** mindestens eine erhöhte lokale Konzentration eines oder mehrerer Nachbarsegmente von mindestens einem Molekül in der Umgebung mindestens eines Nachbarsegments mindestens eines anderen Moleküls in Form einer entropischen Wechselwirkung korrigiert wird, wobei die mindestens zwei Moleküle von derselben oder unterschiedlichen chemischen Spezies sein können.

4. Verfahren nach einem der vorherigen Ansprüche, das ein Berechnen von Folgendem beinhaltet:

$$g_{\mu v}^{lce} = -k_B T f_{corr} \left[ \ln \left\{ \frac{(1 - \widetilde{\Theta}_\mu^{lid}) \Theta^v + \widetilde{\Theta}_\mu^v}{\Theta^v} \right\} + \ln \left\{ \frac{(1 - \widetilde{\Theta}_v^{lid}) \Theta^\mu + \widetilde{\Theta}_v^\mu}{\Theta^\mu} \right\} \right],$$

   wobei:

   > $k_B$ die Boltzmann-Konstante ist;
   > T die Temperatur ist;
   > $\Theta^v$ der Oberflächenanteil von Segmenttyp $v$ ist;
   >
   > $\widetilde{\Theta}_\mu^v$ ein Annäherungswert der induzierten Konzentration von Typensegment $v$ in der Nähe des Segmenttyps $\mu$ ist;

$$\widetilde{\Theta}_{\mu}^{tot} = \sum_{v} \widetilde{\Theta}_{\mu}^{v}$$ die Summe aller induzierten Konzentrationen an dem Segmenttyp $\mu$ ist; und

$f_{corr}$ ein Faktor ist, wobei insbesondere der Faktor $f_{corr}$ ½ ist, um eine Doppelzählung zu vermeiden, was insbesondere in folgender Berechnung resultiert:

$$g_{\mu v}^{kc} = -\frac{k_B T}{2}\left[\ln\left\{\frac{\Theta^v + \widetilde{\Theta}_{\mu}^v}{(1 + \widetilde{\Theta}_{\mu}^{tot})\Theta^v}\right\} + \ln\left\{\frac{\Theta^{\mu} + \widetilde{\Theta}_{v}^{\mu}}{(1 + \widetilde{\Theta}_{v}^{tot})\Theta^{\mu}}\right\}\right]$$

5. Verfahren nach einem der vorherigen Ansprüche, **dadurch gekennzeichnet, dass** freie Energie von mindestens einer chemischen Spezies in dem System berechnet wird.

6. Verfahren nach einem der vorherigen Ansprüche, **dadurch gekennzeichnet, dass** das chemische Potenzial in Bezug auf die lokale Konzentrationswechselwirkung des mindestens einen Nachbarsegments der einen oder mehreren chemischen Spezies in der Umgebung des mindestens einen Nachbarsegments der gleichen oder einer anderen chemischen Spezies korrigiert wird.

7. Verfahren nach einem der vorherigen Ansprüche, insbesondere nach Anspruch 5, das ein Berechnen des chemischen Potenzials $\mu_i$ für jede Spezies $i$ durch Berechnen von Folgendem beinhaltet:

$$\mu_i = \frac{\partial G}{\partial N_i} = \frac{\partial}{\partial N_i}\left(\frac{N}{2}\sum_{\mu v}\Theta^{\mu}\Theta^v\Psi_{\mu v}\left[g_{\mu v}(x) + \ln\Psi_{\mu v}\right]\right)$$

$$= \mu_i^0 + \frac{N}{2}\sum_{\mu v}\Theta^{\mu}\Theta^v\Psi_{\mu v}\left[\frac{\partial g_{\mu v}(x)}{\partial N_i}\right] = \mu_i^0 + \frac{N}{2}\sum_{\mu v}p_{\mu v}\left[\frac{\partial g_{\mu v}(x)}{\partial N_i}\right] \equiv \mu_i^0 + \mu_i^{corr}$$

wobei:

$G$ die gesamte freie Energie des Systems ist, wobei

$$G = \frac{N}{2}\sum_{\mu v}\Theta^v\Theta^{\mu}\Psi_{\mu v}[g_{\mu v} + \ln\Psi_{\mu v}]$$

$N_i$ die Anzahl an Molekülen von Spezies $i$ ist;
$N$ die Anzahl an Molekülen in dem System ist;
$\Theta^v$ der Oberflächenanteil von Segmenttyp $v$ ist;
$\mu v$ ein Paar von Segmenttypen $\mu$ und $v$ bezeichnet;
$p_{\mu v}$ die Segmentberührungswahrscheinlichkeit für das Paar $\mu v$ ist, wobei $p_{\mu v} = \Theta^{\mu}\Psi_{\mu v}\Theta^v$; und
$g_{\mu v}$ die Wechselwirkungsenergie für das Paar $\mu v$ ist.

8. Verfahren nach einem der vorherigen Ansprüche, **dadurch gekennzeichnet, dass** mindestens einer der Berechnungsschritte mindestens einmal wiederholt wird, vorzugsweise in einer 2. oder höheren Schleife.

9. Rechnervorrichtung zur Simulation von mindestens einer physikalischen Eigenschaft eines Systems aus einer oder mehreren chemischen Spezies, das mindestens eine chemische Spezies beinhaltet, die in mindestens einer chemischen Spezies gelöst ist, unter Verwendung einer zweiten quasi-chemischen, COSMO-RS-, COSMO-SAC- oder COSMOSPACE-Berechnung, die zusammensetzungsabhängige Segmentwechselwirkungen $g_{\mu v}$ involviert und mit Gibbs-Duhem konsistente chemische Potentiale und Aktivitätskoeffizienten oder mit Gibbs-Duhem konsistente chemische Potentiale oder mit Gibbs-Duhem konsistente Aktivitätskoeffizienten erreicht, wobei die Rechnervorrichtung konfiguriert ist, um das Verfahren nach mindestens einem der vorherigen Ansprüche 1 bis 8 durchzuführen.

**Revendications**

1. Procédé de sélection de solvants pour une étape de réaction faisant appel à un procédé mis en œuvre par ordinateur pour la simulation d'au moins une propriété physique d'un système d'une ou plusieurs espèces chimiques qui comprend au moins une espèce chimique dissoute dans au moins une espèce chimique, à l'aide d'un deuxième calcul quasi-chimique, COSMO-RS, COSMO-SAC ou COSMOSPACE impliquant des interactions de segments dépendantes de la composition $g_{\mu v}$ et permettant d'obtenir des potentiels chimiques et des coefficients d'activité cohérents avec la relation de Gibbs-Duhem, ou des potentiels chimiques cohérents avec la relation de Gibbs-Duhem, ou des coefficients d'activité cohérents avec la relation de Gibbs-Duhem, **caractérisé en ce que** les probabilités de contact de segments, résultant d'un premier calcul quasi-chimique, COSMO-RS, COSMO-SAC ou COSMOSPACE pour quantifier les concentrations locales induites de types de surface ou de densités de charge de polarisation dues aux segments voisins de segments voisins en contact, sont utilisées comme variable d'entrée dans le deuxième calcul, le deuxième calcul aboutissant ainsi à des probabilités de contact de segments modifiées qui reflètent les corrélations locales des polarités de surface, dans lequel les concentrations locales induites sont converties en une correction entropique pour l'interaction par paires de segments et le deuxième calcul utilise les énergies d'interaction corrigées en fonction de la concentration locale.

**2.** Procédé selon la revendication 1, dans lequel le procédé pour la simulation comprend le fait de calculer le potentiel chimique $\mu_i$ de chaque espèce $i$ en calculant l'équation suivante :

$$\mu_i^* = \mu_i^0 + \mu_i^{*corr} = k_B T \sum_{v \in i} \ln \gamma_*^v + \frac{N}{2} \sum_{\mu v} p_{\mu v}^* \left[ \frac{\partial g_{\mu v}}{\partial N_i} \right]$$

où :

$k_B$ est la constante de Boltzmann ;
T est la température ;
$v \, \varepsilon \, i$ désigne l'ensemble de types de segments v sur l'espèce $i$ ;
$\gamma_*^v$ est le coefficient d'activité de segment non corrigé du type de segment $v$ ;
$N$ est le nombre de molécules dans le système ;
$N_i$ est le nombre de molécules de l'espèce $i$ ;
$\mu v$ désigne une paire de types de segments $\mu$ et $v$ ;
$g_{\mu v}$ est l'énergie d'interaction pour la paire $\mu v$ ; et
$p_{\mu v}^*$ est la probabilité de contact de segments pour les types de segments $\mu$ et $v$.

**3.** Procédé selon l'une quelconque des revendications précédentes, **caractérisé en ce qu'**au moins une concentration locale accrue d'un ou plusieurs segments voisins d'au moins une molécule dans l'environnement d'au moins un segment voisin d'au moins une autre molécule est corrigée sous la forme d'une interaction entropique, dans laquelle les au moins deux molécules peuvent être de la même espèce chimique ou d'espèces chimiques différentes.

**4.** Procédé selon l'une quelconque des revendications précédentes, comprenant le fait de calculer :

$$g_{\mu v}^{lcc} = -k_B T f_{corr} \left[ \ln\left\{ \frac{(1 - \widetilde{\Theta}_\mu^{tot})\Theta^v + \widetilde{\Theta}_\mu^v}{\Theta^v} \right\} + \ln\left\{ \frac{(1 - \widetilde{\Theta}_v^{tot})\Theta^\mu + \widetilde{\Theta}_v^\mu}{\Theta^\mu} \right\} \right],$$

où :

$k_B$ est la constante de Boltzmann ;
T est la température ;
$\Theta^v$ est la fraction de surface du type de segment $v$ ;
$\widetilde{\Theta}_\mu^v$ est une approximation de la concentration induite du type de segment $v$ à proximité du type de segment $\mu$ ;
$\widetilde{\Theta}_\mu^{tot} = \sum_v \widetilde{\Theta}_\mu^v$ est la somme de toutes les concentrations induites au niveau du type de segment $\mu$ ; et
$f_{corr}$ est un facteur, en particulier dans lequel le facteur $f_{corr}$ est ½, évitant le double comptage, se traduisant en particulier par le calcul suivant :

$$g_{\mu\nu}^{lcc} = -\frac{k_B T}{2}\left[\ln\left\{\frac{\Theta^{\nu}+\widetilde{\Theta}_{\mu}^{\nu}}{\left(1+\widetilde{\Theta}_{\mu}^{tot}\right)\Theta^{\nu}}\right\} + \ln\left\{\frac{\Theta^{\mu}+\widetilde{\Theta}_{\nu}^{\mu}}{\left(1+\widetilde{\Theta}_{\nu}^{tot}\right)\Theta^{\mu}}\right\}\right]$$

5. Procédé selon l'une quelconque des revendications précédentes, **caractérisé en ce que** l'énergie libre d'au moins une espèce chimique dans le système est calculée.

6. Procédé selon l'une quelconque des revendications précédentes, **caractérisé en ce que** le potentiel chimique par rapport à l'interaction en fonction de la concentration locale d'au moins un segment voisin de la ou des espèces chimiques dans l'environnement de l'au moins un segment voisin de la même espèce chimique ou d'une autre espèce chimique est corrigé.

7. Procédé selon l'une quelconque des revendications précédentes, en particulier selon la revendication 5, comprenant le fait de calculer le potentiel chimique $\mu_i$ de chaque espèce $i$ en calculant :

$$\mu_i = \frac{\partial G}{\partial N_i} = \frac{\partial}{\partial N_i}\left(\frac{N}{2}\sum_{\mu\nu}\Theta^{\mu}\Theta^{\nu}\Psi_{\mu\nu}\left[g_{\mu\nu}(x)+\ln\Psi_{\mu\nu}\right]\right)$$

$$= \mu_i^0 + \frac{N}{2}\sum_{\mu\nu}\Theta^{\mu}\Theta^{\nu}\Psi_{\mu\nu}\left[\frac{\partial g_{\mu\nu}(x)}{\partial N_i}\right] = \mu_i^0 + \frac{N}{2}\sum_{\mu\nu}p_{\mu\nu}\left[\frac{\partial g_{\mu\nu}(x)}{\partial N_i}\right] \equiv \mu_i^0 + \mu_i^{corr}$$

où :

G est l'énergie libre totale du système, avec $G = \frac{N}{2}\sum_{\mu\nu}\Theta^{\nu}\Theta^{\mu}\Psi_{\mu\nu}\left[g_{\mu\nu} + \ln\Psi_{\mu\nu}\right]$

$N_i$ est le nombre de molécules de l'espèce $i$ ;
N est le nombre de molécules dans le système ;
$\Theta^{\nu}$ est la fraction de surface du type de segment $\nu$ ;
$\mu\nu$ désigne une paire de types de segments $\mu$ et $\nu$ ;
$p_{\mu\nu}$ est la probabilité de contact de segments pour la paire $\mu\nu$, avec $p_{\mu\nu} = \Theta^{\mu}\Psi_{\mu\nu}\Theta^{\nu}$ ; et
$g_{\mu\nu}$ est l'énergie d'interaction pour la paire $\mu\nu$.

8. Procédé selon l'une quelconque des revendications précédentes, **caractérisé en ce qu'**au moins une des étapes de calcul sera répétée au moins une fois, de préférence dans une 2ième boucle ou une boucle supérieure.

9. Appareil informatique pour la simulation d'au moins une propriété physique d'un système d'une ou plusieurs espèces chimiques qui comprend au moins une espèce chimique dissoute dans au moins une espèce chimique, à l'aide d'un calcul quasi-chimique, COSMO-RS, COSMO-SAC ou COSMOSPACE impliquant des interactions de segments dépendantes de la composition $g_{\mu\nu}$ et permettant d'obtenir des potentiels chimiques et des coefficients d'activité cohérents avec la relation de Gibbs-Duhem, ou des potentiels chimiques cohérents avec la relation de Gibbs-Duhem, ou des coefficients d'activité cohérents avec la relation de Gibbs-Duhem, dans lequel l'appareil informatique est configuré pour mettre en œuvre le procédé selon au moins l'une des revendications 1 à 8 précédentes.

Fig. 1

Fig. 2

Fig. 3

Fig. 4

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Non-patent literature cited in the description

- **E.A. GUGGENHEIM**. Mixtures. Oxford University Press, 1952
- **KLAMT, A**. Conductor-like Screening Model for Real Solvents: A New Approach to the Quantitative Calculation of Solvation Phenomena. *J. Phys. Chem*, 1995, vol. 99 (7), 2224-2235
- **KLAMT, A** ; **JONAS, V.** ; **BÜRGER, T.** ; **LOHRENZ, J. C**. Refinement and Parametrization of COSMO-RS. *J. Phys. Chem. A*, 1998, vol. 102 (26), 5074-5085
- **KLAMT, A**. COSMO-RS From Quantum Chemistry to Fluid Phase Thermodynamics and Drug Design. Elsevier, 2005
- **KLAMT, A** ; **SCHÜÜRMANN, G.** COSMO: A New Approach to Dielectric Screening in Solvents with Explicit Expressions for the Screening Energy and Its Gradient. *J. Chem. Soc. Perkin Trans. 2*, 1993, vol. 1993 (5), 799-805
- **KLAMT, A.** ; **KROOSHOF, G. J. P** ; **TAYLOR, R**. COSMOSPACE: Alternative to Conventional Activity-Coefficient Models. *AIChE J.*, 2002, vol. 48 (10), 2332-2349
- **KLAMT, A.** ; **ECKERT, F.** ; **ARLT, W**. COSMO-RS: An Alternative to Simulation for Calculating Thermodynamic Properties of Liquid Mixtures. *Annu. Rev. Chem. Biomol. Eng.*, 2010, vol. 1 (1), 101-122
- **LUCAS, K**. Molecular Models for Fluids. Cambridge University Press, 2007
- **GUGGENHEIM, E. A** ; **TURGEON, J. C.** *Specific Interaction of Ions. Trans. Faraday Soc.*, 1955, vol. 51, 747-761
- **EGNER, K** ; **GAUBE, J** ; **PFENNIG, A**. GEQUAC, an Excess Gibbs Energy Model Describing Associating and Nonassociating Liquid Mixtures by a New Model Concept for Functional Groups. *Fluid Phase Equilibria*, 1999, vol. 158, 381-389
- **ABRAMS, D. S.** ; **PRAUSNITZ, J. M**. Statistical Thermodynamics of Liquid Mixtures: A New Expression for the Excess Gibbs Energy of Partly or Completely Miscible Systems. *AIChE J.*, 1975, vol. 21 (1), 116-128
- **FREDENSLUND, A** ; **JONES, R. L** ; **PRAUSNITZ, J. M**. Group-Contribution Estimation of Activity Coefficients in Nonideal Liquid Mixtures. *AIChE J.*, 1975, vol. 21 (6), 1086-1099
- **LARSEN, B. L.** ; **RASMUSSEN, P**. A Comparison between the Quasichemical Model and Two-Fluid Local-Composition Models. *Fluid Phase Equilibria*, 1986, vol. 28 (1), 1-11
- **LIN, S.-T.** ; **SANDLER, S. I.** A Priori Phase Equilibrium Prediction from a Segment Contribution Solvation Model. *Ind. Eng. Chem. Res.*, 2002, vol. 41 (9), 2332-2334
- **SACHSENHAUSER, T.** ; **REHFELDT, S.** ; **KLAMT, A.** ; **ECKERT, F** ; **KLEIN, H.** Consideration of Dimerization for Property Prediction with COSMO-RS-DARE. *Fluid Phase Equilibria*, 2014, vol. 382, 89-99